# EUROPEAN PATENT APPLICATION

(11) **EP 4 295 812 A1**
(43) Date of publication of application: **27.12.2023**
(21) Application number: 22756431.7
(22) Date of filing: 11.02.2022
(51) Int. Cl.: A61F 2/12, A61F 2/00, A61L 27/18, A61L 27/50

(54) **ARTIFICIAL IMPLANT AND METHOD FOR MANUFACTURING SAME**

(30) Priority: 18.02.2021 KR 20210022039; 25.02.2021 KR 20210025218
(71) Applicant: Osstemimplant Co., Ltd., Gangseo-gu Seoul 07789 (KR)
(72) Inventor: SIM, Eun Jung, Seoul 07568 (KR); KIM, Byung Hwi, Yongin-si, Gyeonggi-do 16968 (KR); KIM, Min Kyoung, Incheon 22765 (KR); SONG, Ju Dong, Busan 48272 (KR); JANG, Il Seok, Yangsan-si, Gyeongsangnam-do 50653 (KR)
(74) Representative: Beck & Rössig European Patent Attorneys
(86) International application number: PCT/KR2022/002062
(87) International publication number: WO 2022/177232

(57) **Abstract**

Disclosed are an artificial implant and a method for manufacturing same, the artificial implant comprising: a silicone shell consisting of an upper portion, a lower portion, and a side portion; and a filler injected into the silicone shell, wherein the silicone shell includes at least one silicone layer and at least one reinforcing layer, the reinforcing layer being provided on at least a portion of the lower portion and the side portion of the silicone shell, or the silicone shell includes a layered structure having a step.

## Description

### [Technical Field]

The present invention relates to an artificial implant having excellent stability and a method of manufacturing the same.

### [Background Art]

Artificial breast implants are Class IV medical devices in which a gel having appropriate viscosity or saline is filled in a silicone shell forming the outer shell of the implant, and require special stability in vivo. In the implant industry, properties required as a medical device have been secured by adjusting the thickness of a silicone shell or changing a filler component, and research and development is being conducted to realize a natural shape by adjusting the properties of a filler.

However, artificial implants still have the potential for post-implantation side effects. Particularly, a rippling phenomenon frequently occurs due to folding of a silicone shell. The rippling phenomenon is a side effect in which wrinkles formed on the implant surface appear as wavy patterns on the skin surface of the breast. This phenomenon may reduce the aesthetic satisfaction of users, and as folding continuously occurs at a specific site of a silicone shell which is the outer shell of implants, durability is degraded at the corresponding site, and thus implant rupture may be caused.

As described above, when artificial implants rupture, a filler present in the implant may leak out. As the filler leaks out, inflammatory reactions may occur inside the human body, and furthermore, tissue necrosis, bacterial infections, capsular contracture, breast implant-associated anaplastic large cell lymphoma (BIA-ALCL), and the like, which may cause extreme pain to users due to overactive immune responses, may be caused. Particularly, users who develop capsular contracture need to undergo a reoperation, and users who develop BIA-ALCL may face the risk of death.

In order to solve the above-described problems of the conventional artificial implant, it is most important to prevent the rupture of implants in advance. Particularly, since the rippling phenomenon continuously occurs at a specific site of a silicone shell, which is the outer shell of implants, to degrade durability, there is a need for the research and development of an artificial implant capable of imparting mechanical properties to the corresponding site and a method of manufacturing the same.

### [Disclosure]

### [Technical Problem]

The present invention is designed to solve the above-described problems of the related art and is directed to providing an artificial implant having excellent mechanical stability and a method of manufacturing the same, and thus rupture is prevented.

### [Technical Solution]

One aspect of the present invention provides an artificial implant, which includes: a silicone shell consisting of an upper portion, a lower portion, and a side portion; and a filler injected into the silicone shell, wherein the silicone shell includes one or more silicone layers and one or more reinforcing layers formed on at least a portion of a surface of the silicone layer, and the reinforcing layer is provided in at least a portion of the lower portion and side portion of the silicone shell.

Another aspect of the present invention provides an artificial implant, which includes: a silicone shell consisting of an upper portion, a lower portion, and a side portion; and a filler injected into the silicone shell, wherein the silicone shell includes one or more silicone layers and one or more reinforcing layers formed on at least a portion of a surface of the silicone layer, and the silicone shell has a layered structure in which a step is formed.

In an embodiment, at least a portion of the silicone shell may be formed by continuously or alternately laminating the one or more silicone layers and the one or more reinforcing layers.

In an embodiment, the silicone shell may have a thickness of 0.4 to 1.2 mm, and the reinforcing layer may have a thickness of 0.01 to 0.3 mm.

In an embodiment, the upper portion and lower portion of the silicone shell may have thicknesses of 0.4 to 0.8 mm, and the reinforced side portion of the silicone shell may have a thickness of 0.4 to 1.2 mm.

In an embodiment, a portion of the silicone shell in which the reinforcing layer is provided may satisfy at least one of the following conditions (i) and (ii):
(i) a rupture strength of 25 to 50 N; and
(ii) an elongation rate of 400 to 520%.

In an embodiment, a cross-section of the artificial implant may have one shape selected from a circular shape, an oval shape, and a teardrop shape.

Still another aspect of the present invention provides a method of manufacturing an artificial implant, which includes the steps of: (a) immersing a breast-shaped mold in a silicone solution to form one or more silicone layers; (b) applying a silicone solution onto at least a portion of the silicone layer to form one or more reinforcing layers; (c) drying and curing the formed silicone layers and reinforcing layers to manufacture a silicone shell; (d) forming an injection hole so that a filler is able to be injected into the silicone shell; and (e) inputting the filler through the injection hole.

In an embodiment, in step (b), the reinforcing layer may be formed in at least a portion of a lower portion and side portion of the silicone shell.

In an embodiment, in step (b), the reinforcing layer may be formed so that the silicone shell has a layered structure having a step.

In an embodiment, steps (a) and (b) may each be repeatedly performed 1 to 10 times.

In an embodiment, steps (a) and (b) may each be alternately performed 1 to 10 times.

### [Advantageous Effects]

According to an aspect, an artificial implant, which is capable of preventing a folding phenomenon and the resulting rupture and breakage by having excellent durability and rupture resistance through reinforcement of mechanical weaknesses, and a method of manufacturing the same can be provided.

However, it is to be understood that the effect of an aspect of the specification is not limited to the above-described effect but include all effects deducible from the configuration described in the detailed description of the specification or in the claims.

### [Description of Drawings]

FIG. 1 shows a cross-sectional view of an artificial implant whose lower portion is reinforced according to one embodiment of the specification.
FIG. 2 shows a cross-sectional view of an artificial implant whose lower portion is reinforced according to another embodiment of the specification.
FIG. 3 shows a cross-sectional view of an artificial implant whose side portion is reinforced according to still another embodiment of the specification.
FIG. 4 shows a cross-sectional view of an artificial implant whose side portion is reinforced according to yet another embodiment of the specification.
FIG. 5 shows a cross-sectional view of an artificial implant whose lower portion and side portion are reinforced according to yet another embodiment of the specification.
FIG. 6 shows a cross-sectional view of an artificial implant whose lower portion and side portion are reinforced according to yet another embodiment of the specification.
FIG. 7 shows a layered structure of the end of a reinforced structure of an artificial implant according to one embodiment of the specification.
FIG. 8 shows a layered structure of the end of a reinforced structure of an artificial implant according to another embodiment of the specification.
FIG. 9 is a graph showing the elongation rates of the examples and comparative example of the specification.
FIG. 10 is a graph showing the rupture strengths of the examples and comparative example of the specification.

### [Modes of the Invention]

Hereinafter, an aspect of the specification will be described with reference to the accompanying drawings. However, it should be understood that the descriptions of the specification can be implemented in various other forms, and that it is not intended to limit the present invention to the exemplary embodiments. Also, in the drawings, descriptions of parts unrelated to the detailed description are omitted to clearly describe an aspect of the specification. Throughout the specification, like numbers refer to like elements.

Throughout the specification, the phrase a certain part is "connected" to another part means that the certain part is not only "directly connected" to the other part but also "indirectly connected" to the other part through another member interposed between the two parts. Also, the phrase a certain part "includes" a certain element means that the certain part may further include, instead of excluding, another element unless particularly indicated otherwise.

When a numerical value is presented in the specification, the value has the precision of significant digits provided in accordance with the standard rules in chemistry for significant digits unless its specific range is stated otherwise. For example, the numerical value 10 includes the range of 5.0 to 14.9 and the numerical value 10.0 includes the range of 9.50 to 10.49.

Hereinafter, an embodiment of the specification will be described in detail with reference to the accompanying drawings.

### Artificial implant

An artificial implant 100 according to one aspect includes: a silicone shell consisting of an upper portion 110, a lower portion 120, and a side portion 130; and a filler injected into the silicone shell, wherein the silicone shell includes one or more silicone layers and one or more reinforcing layers, and the reinforcing layer is provided in at least a portion of the lower portion 120 and side portion 130 of the silicone shell. The reinforcing layer provided in at least a portion of the silicone shell may form a reinforced structure 140 to improve the stability of the artificial implant 100.

An artificial implant 100 according to another aspect includes: a silicone shell consisting of an upper portion 110, a lower portion 120, and a side portion 130; and a filler injected into the silicone shell, wherein the silicone shell includes one or more silicone layers and one or more reinforcing layers formed on at least a portion of a surface of the silicone layer, and the silicone shell has a layered structure in which a step is formed. The reinforcing layers formed on at least a portion of a surface of the silicone layer may form a reinforced structure 140 to improve the stability of the artificial implant 100.

A conventional artificial implant is manufactured by forming a silicone shell having the same thickness in all portions and then injecting a filler thereinto. When the conventional artificial implant is inserted into the human body, the folding phenomenon of a shell may easily occur at a specific area due to the properties of a gel and gravity. For example, in the case of the conventional artificial implant, the folding phenomenon of a shell frequently occurs at a side portion. When the folding phenomenon of a shell occurs while the artificial implant is inserted into the body, a rippling phenomenon in which wave patterns are revealed on the surface of the breast is caused, and the properties of a side portion are degraded, and thus a silicone shell may be easily broken. The artificial implant 100 according to an aspect of the specification can improve the above problems by having reinforced durability and rupture resistance.

The silicone shell may be basically formed by laminating one or more silicone layers. In an example, at least a portion of the lower portion 120 and the side portion 130 may be formed by laminating one or more silicone layers and one or more reinforcing layers. A filler may be injected into the silicone shell to maintain the shape of the artificial implant 100.

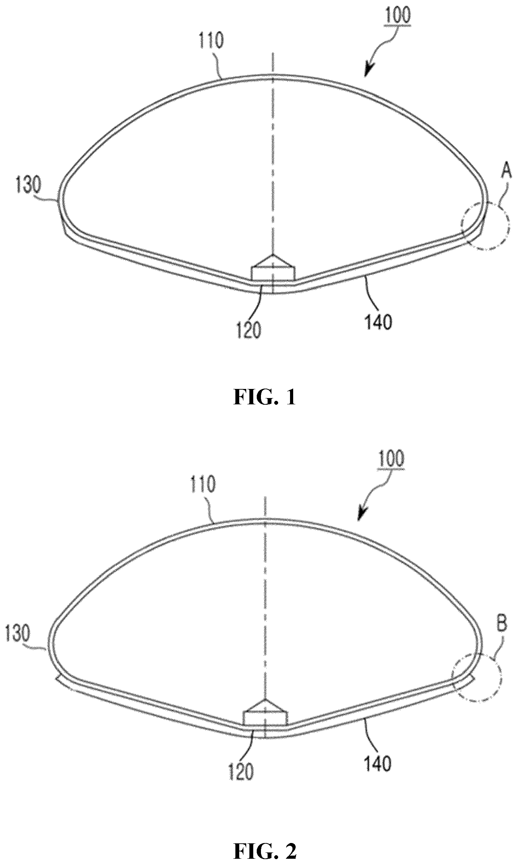

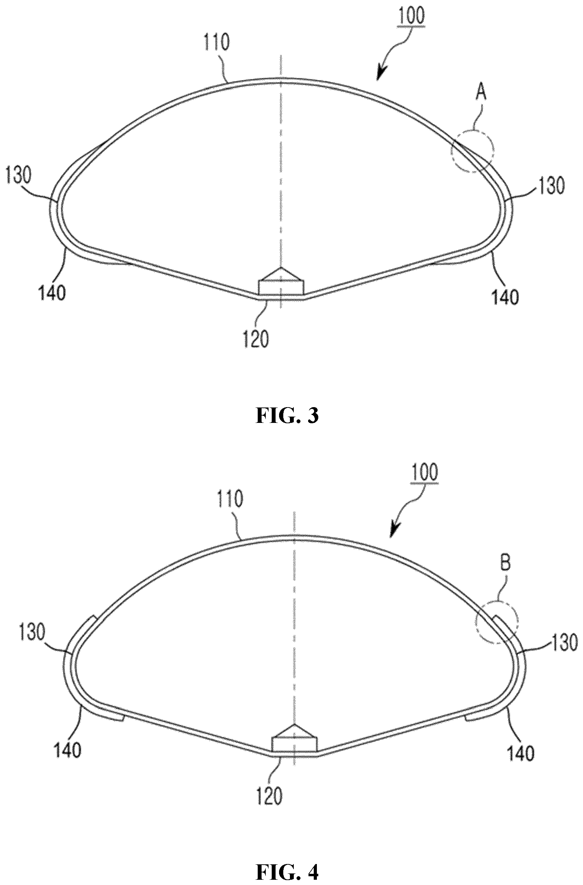

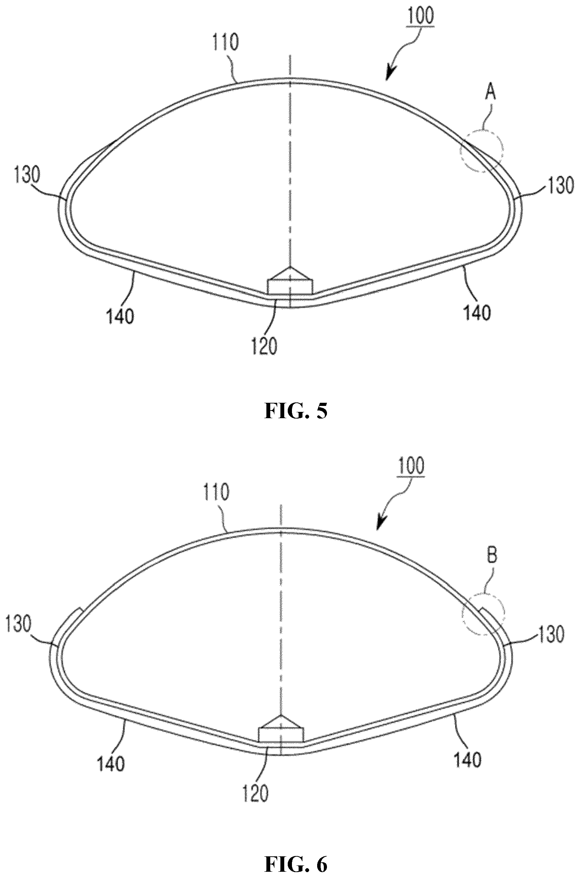

In an example, FIGS. 1 and 2 show that the reinforced structure 140 is formed by providing the reinforcing layer in the lower portion 120 of the silicone shell of the artificial implant 100, FIGS. 3 and 4 show that the reinforced structure 140 is formed by providing the reinforcing layer in the side portion 130 of the silicone shell of the artificial implant 100, and FIGS. 5 and 6 show that the reinforced structure 140 is formed by providing the reinforcing layer in the lower portion 120 and side portion 130 of the silicone shell of the artificial implant 100. As described above, the artificial implant 100 may have the reinforced structure 140 in which one or more reinforcing layers are provided in a portion of the silicone shell according to the required purpose. Due to the reinforced structure 140, the durability and rupture resistance of the artificial implant 100 are improved, and thus a folding phenomenon can be prevented.

The end of the reinforced structure 140 may have a layered structure having a step. In a non-limiting example, the silicone shell may include 1 to 10 reinforcing layers, for example, 1, 2, 3, 4, 5, 6, 7, 8, 9, 10 reinforcing layers, or a number in a range between two of the above values. The layered structure may vary depending on the desired artificial implant 100 product. In an example, when a stepped multilayer structure having a step is included, stress concentration is prevented, and thus usability can be improved. In another example, when a monolayer structure having a step is included, the strength of a reinforced site can be uniformly improved. The reinforced structure 140 may be formed in at least a portion of the upper portion, lower portion, and side portion of the silicone shell.

FIGS. 1, 3, and 5 show a gradual layered structure A in which two or more layers are formed stepwise. When a gradual layered structure having a gentle end without a sharp difference in thickness is formed, stress concentration is prevented, and thus usability can be improved

FIGS. 2, 4, and 6 show a layered structure B in which one layer is formed stepwise. When a layered structure having a step is formed, the strength of a site to be reinforced can be uniformly improved.

The one or more silicone layers and one or more reinforcing layers forming the above-described reinforced structure 140 of the silicone shell may be continuously or alternately formed. The reinforced structure 140 may be freely selected according to the desired features of a final product.

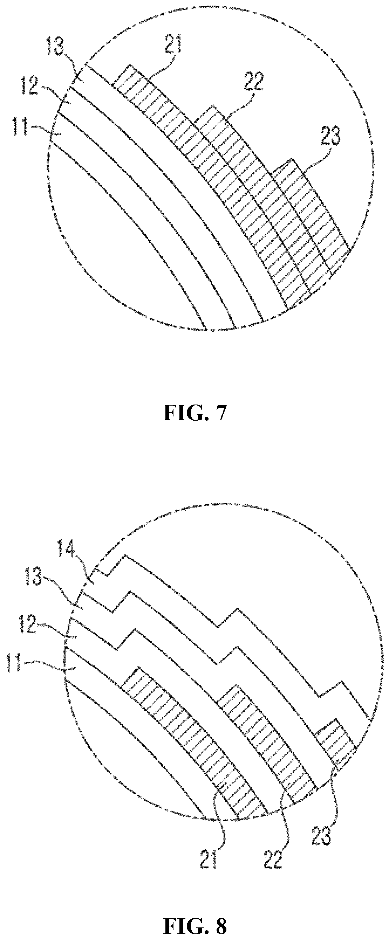

For example, FIG. 7 shows a structure in which silicone layers and reinforcing layers are each continuously formed. In other words, a first silicone layer 11, a second silicone layer 12, a third silicone layer 13, a first reinforcing layer 21, a second reinforcing layer 22, and a third reinforcing layer 23 may be sequentially formed to manufacture an artificial implant having the structure of FIG. 7. The structure may be relatively easily manufactured.

As another example, FIG. 8 shows a structure in which silicone layers and reinforcing layers are alternately formed. In other words, a first silicone layer 11, a first reinforcing layer 21, a second silicone layer 12, a second reinforcing layer 22, a third silicone layer 13, a third reinforcing layer 23, and a fourth silicone layer 14 may be sequentially formed to manufacture an artificial implant having the structure of FIG. 8. Since the structure is formed so that the end of each reinforcing layer is covered with a silicone layer, the reinforcing layer can be prevented from being detached.

In the case of the artificial implant, the thicknesses of all portions may be uniform, or a portion in which the reinforced structure 140 is provided may be thicker than the remaining portion. When the thicknesses of all portions are uniform, a component of the reinforcing layer may vary to improve the mechanical stability of the artificial implant. When the thicknesses according to portions are different, components of the silicone layer and the reinforcing layer may be the same or different according to the purpose.

The silicone shell may have a thickness of 0.4 to 1.2 mm, and the reinforcing layer may have a thickness of 0.01 to 0.3 mm. In an example, a portion in which the reinforced structure 140 is not provided may have a thickness of 0.4 to 0.8 mm, for example, 0.4 mm, 0.5 mm, 0.6 mm, 0.7 mm, 0.8 mm, or a thickness in a range between two of the above values, but the present invention is not limited thereto. In this case, a portion in which the reinforced structure 140 is provided may have a thickness of 0.4 to 1.2 mm, for example, 0.4 mm, 0.5 mm, 0.6 mm, 0.7 mm, 0.8 mm, 0.9 mm, 1.0 mm, 1.1 mm, 1.2 mm, or a thickness in a range between two of the above values, but the present invention is not limited thereto. When the thickness of the reinforced structure 140 of the silicone shell is less than 0.4 mm, mechanical properties are degraded, and thus the artificial implant 100 may break and aesthetic side effects may occur, and when the thickness thereof exceeds 1.2 mm, users who have received the artificial implant 100 may feel discomfort such as a foreign body sensation. The thickness may encompass the thickness of the silicone layer or the thicknesses of the silicone layer and the reinforcing layer according to a portion. The reinforcing layer may have a thickness of 0.01 to 0.3 mm, for example, 0.01 mm, 0.02 mm, 0.03 mm, 0.04 mm, 0.05 mm, 0.06 mm, 0.07 mm, 0.08 mm, 0.09 mm, 0.1 mm, 0.11 mm, 0.12 mm, 0.13 mm, 0.14 mm, 0.15 mm, 0.16 mm, 0.17 mm, 0.18 mm, 0.19 mm, 0.2 mm, 0.21 mm, 0.22 mm, 0.23 mm, 0.24 mm, 0.25 mm, 0.26 mm, 0.27 mm, 0.28 mm, 0.29 mm, 0.3 mm, or a thickness in a range between two of the above values, but the present invention is not limited thereto. When the thickness of the reinforcing layer is less than 0.01 mm, the stability of the reinforced structure 140 may be degraded, and when the thickness thereof exceeds 0.3 mm, a thickness deviation between the portions of the silicone shell is excessively generated, and thus durability may be degraded due to stress concentration. The thickness of each reinforcing layer may vary depending on the thickness of the reinforced structure 140 and the number of layers forming the reinforced structure 140. When many relatively thin reinforcing layers are laminated, the user's foreign body sensation can be minimized, and when a small number of thick reinforcing layers are used, convenience in preparation can be excellent.

In another example, the upper portion 110 and lower portion 120 of the silicone shell may have thicknesses of 0.4 to 0.8 mm, for example, 0.4 mm, 0.5 mm, 0.6 mm, 0.7 mm, 0.8 mm, or thicknesses in a range between two of the above values, but the present invention is not limited thereto. When the thicknesses of the upper portion 110 and lower portion 120 of the silicone shell are less than 0.4 mm, the durability of the silicone shell is degraded, and thus rupture may occur even due to small stimuli, and when the thicknesses thereof exceed 0.8 mm, users who received the artificial implant 100 may feel discomfort such as a foreign body sensation. Also, the reinforced side portion 130 of the silicone shell may have a thickness of 0.4 to 1.2 mm, for example, 0.4 mm, 0.5 mm, 0.6 mm, 0.7 mm, 0.8 mm, 0.9 mm, 1.0 mm, 1.1 mm, 1.2 mm, or a thickness in a range between two of the above values, but the present invention is not limited thereto. When the thickness of the side portion 130 of the silicone shell is less than 0.4 mm, the properties of the side portion 130 of the silicone shell are degraded, and thus the artificial implant 100 may break and aesthetic side effects may occur, and when the thickness thereof exceeds 1.2 mm, users who received the artificial implant 100 may feel discomfort such as a foreign body sensation due to the excessively thick side portion 130. The thickness of the reinforced side portion may encompass the thickness of the silicone layer and the thickness of the reinforced structure 140.

Meanwhile, a portion of the silicone shell in which the reinforcing layer is provided may satisfy at least one of the following conditions (i) and (ii):
(i) a rupture strength of 25 to 50 N; and
(ii) an elongation rate of 400 to 520%.

For example, the portion of the silicone shell in which the reinforcing layer is provided may have a rupture strength of 25 N, 26 N, 27 N, 28 N, 29 N, 30 N, 31 N, 32 N, 33 N, 34 N, 35 N, 36 N, 37 N, 38 N, 39 N, 40 N, 41 N, 42 N, 43 N, 44 N, 45 N, 46 N, 47 N, 48 N, 49 N, 50 N, or a rupture strength in a range between two of the above values, but the present invention is not limited thereto. In the case of the silicone shell constituting the outer shell of the artificial implant 100, durability and rupture resistance may be reinforced by the reinforcing layer, and accordingly, the rupture strength may satisfy a range of 25 to 50 N.

In addition, the portion of the silicone shell in which the reinforcing layer is provided may have, for example, an elongation rate of 400%, 410%, 420%, 430%, 440%, 450%, 460%, 470%, 480%, 490%, 500%, 510%, 520%, or an elongation rate in a range between two of the above values, but the present invention is not limited thereto. When the elongation rate is less than 400%, the texture and tactile sensation of the silicone shell are degraded, and thus user preference may be degraded. When the elongation rate exceeds 520%, the artificial implant 100 may easily break or rupture.

The artificial implant may have varying shapes depending on the insertion site or the purpose of the procedure and may be inserted into various body parts such as the breast, nose, buttocks, forehead, and the like, but the shape thereof is not particularly limited.

In a non-limiting example, the artificial implant 100 may serve as an implant in the body by injecting a filler into the silicone shell. The filler may be a silicone gel, and the silicone gel may be polyorganosiloxane. The polyorganosiloxane may be one selected from the group consisting of dimethylsiloxane, methyl hydrogen siloxane, methylphenylsiloxane, diphenylsiloxane, dimethylvinylsiloxane, trifluoropropylsiloxane, and a mixture of two or more thereof, but the type thereof is not particularly limited as long as it can realize the functional characteristics of an artificial implant.

The cross-section of the artificial implant may have one shape selected from a circular shape, an oval shape, and a teardrop shape, but the present invention is not limited thereto. Generally, since users have different breast shapes, the shape of the artificial implant may be selected in consideration of an original shape and user preferences such as a desired shape, a desired size, and the like.

### Method of manufacturing artificial implant

A method of manufacturing an artificial implant according to another aspect includes the steps of: (a) immersing a breast-shaped mold in a silicone solution to form one or more silicone layers; (b) applying a silicone solution onto at least a portion of the silicone layer to form one or more reinforcing layers; (c) drying and curing the formed silicone layers and reinforcing layers to manufacture a silicone shell; (d) forming an injection hole so that a filler is able to be injected into the silicone shell; and (e) inputting the filler through the injection hole.

Through steps (a) to (c), a silicone shell forming the outer shell of the artificial implant may be manufactured. As described above, the silicone shell may consist of an upper portion 110, a lower portion 120, and a side portion 130 and include one or more silicone layers and one or more reinforcing layers, wherein the reinforcing layer is provided in at least a portion of the lower portion and side portion of the silicone shell, or the silicone shell has a layered structure in which a step is formed. Therefore, a reinforced structure 140 whose thickness is reinforced may be included, and accordingly, the stability of the artificial implant may be improved.

For example, the reinforcing layer in step (b) may be formed in at least a portion of the lower portion and side portion of the silicone shell or formed so that the silicone shell has a layered structure having a step, and as a result, such an artificial implant may be manufactured. Also, the layered structure having a step may be formed in at least a portion of the upper portion, lower portion, and side portion of the silicone shell.

Steps (a) and (b) may each be independently repeatedly performed 1 to 10 times. For example, step (a) may be repeatedly performed 1, 2, 3, 4, 5, 6, 7, 8, 9, or 10 times, and 1, 2, 3, 4, 5, 6, 7, 8, 9, or 10 silicone layers may be laminated according to the number of repetitions of step (a). Also, step (b) may be repeatedly performed 1, 2, 3, 4, 5, 6, 7, 8, 9, or 10 times, and 1, 2, 3, 4, 5, 6, 7, 8, 9, or 10 reinforcing layers may be laminated according to the number of repetitions of step (b), but the present invention is not limited thereto. For example, an artificial implant manufactured by repeatedly performing each of step (a) and step (b) may be manufactured in the form shown in FIG. 7. Referring to FIG. 7, in the case of the artificial implant, a first silicone layer 11, a second silicone layer 12, and a third silicone layer 13 may be sequentially formed, and a first reinforcing layer 21, a second reinforcing layer 22, and a third reinforcing layer 23 may be sequentially formed on at least a portion of the surface of the third silicone layer 13. Like the first to third reinforcing layers 21, 22, and 23 of FIG. 7, when reinforcing layers are formed to have a layered structure in which a gentle step is formed, a gradual layered structure is formed, and thus stress concentration may be further alleviated. When reinforcing layers are formed without any step (not shown), an artificial implant having a reinforced structure 140 may be simply manufactured without any additional manipulation when each layer is formed.

Meanwhile, steps (a) and (b) may be alternately performed 1 to 10 times. For example, an artificial implant manufactured by alternately performing step (a) 4 times and step (b) 3 times may be manufactured in the form shown in FIG. 8. Referring to FIG. 8, in the case of the artificial implant, as step (a) and step (b) are alternately performed, a first silicone layer 11, a first reinforcing layer 21, a second silicone layer 12, a second reinforcing layer 22, a third silicone layer 13, a third reinforcing layer 23, and a fourth silicone layer 14 may be sequentially formed. When the silicone layer and the reinforcing layer are alternately formed as described above, a layered structure in which a step is formed may be easily implemented.

In this way, the number of repetitions of steps (a) to (c) may be adjusted to control the thickness of a layered structure, and a portion to be immersed and coated may be adjusted to form the reinforced structure 140 in at least a portion of the upper portion 110, the lower portion 120, and the side portion 130.

In step (d), an injection hole may be formed so that a filler is able to be injected into the silicone shell. The injection hole may allow a filler to be injected into the silicone shell and serve to prevent the injected filler from leaking out by being closed after the injection.

In step (e), the filler may be input through the injection hole to manufacture the artificial implant. In a non-limiting example, the artificial implant may serve as an implant in the body by injecting the filler into the silicone shell. The filler may be a silicone gel, and the silicon gel may be polyorganosiloxane. The polyorganosiloxane may be one selected from the group consisting of dimethylsiloxane, methyl hydrogen siloxane, methylphenylsiloxane, diphenylsiloxane, dimethylvinylsiloxane, trifluoropropylsiloxane, and a mixture of two or more thereof, but the type thereof is not particularly limited as long as it can realize the functional characteristics of an artificial implant. Also, the insertion site, shape, cross-section, and the like of the artificial implant may be selected in consideration of user preferences such as a desired shape, a desired size, and the like, and a detailed description thereof is the same as described above.

Hereinafter, examples of the specification will be described in further detail. However, the following experimental results are obtained from only a few selected examples of the invention, and the scope and contents of the specification should not be interpreted as being reduced or limited by the few selected examples. The effects of each of the various embodiments of the specification, which are not explicitly set forth below, will be described in detail in relevant sections.

### Example 1

A breast-shaped mold was repeatedly immersed in a silicone solution three times to sequentially form a first silicone layer, a second silicone layer, and a third silicone layer with a uniform thickness of 0.5 mm. A silicone solution was applied onto a side portion of the third silicone layer to laminate a first reinforcing layer, and thus a silicone shell in which a step was formed was manufactured. A filler was injected into the manufactured silicone shell to manufacture an artificial implant.

### Example 2

A breast-shaped mold was repeatedly immersed in a silicone solution three times to sequentially form a first silicone layer, a second silicone layer, and a third silicone layer with a uniform thickness of 0.5 mm. A silicone solution was applied onto a side portion of the third silicone layer to laminate a first reinforcing layer and a second reinforcing layer, and thus a silicone shell in which a step was formed was manufactured. A filler was injected into the manufactured silicone shell to manufacture an artificial implant.

### Example 3

A breast-shaped mold was repeatedly immersed in a silicone solution three times to sequentially form a first silicone layer, a second silicone layer, and a third silicone layer with a uniform thickness of 0.5 mm. A silicone solution was applied onto a side portion of the third silicone layer to laminate a first reinforcing layer, a second reinforcing layer, and a third reinforcing layer, and thus a silicone shell in which a step was formed was manufactured. A filler was injected into the manufactured silicone shell to manufacture an artificial implant.

### Example 4

A breast-shaped mold was immersed in a silicone solution to form a first silicone layer. A silicone solution was applied onto a side portion of the formed first silicone layer to laminate a first reinforcing layer, and then the resultant was immersed in a silicone solution again to form a second silicone layer. As a result, a silicone shell in which a step was formed was manufactured. A filler was injected into the manufactured silicone shell to manufacture an artificial implant.

### Example 5

A breast-shaped mold was immersed in a silicone solution to form a first silicone layer. A silicone solution was applied onto a side portion of the formed first silicone layer to laminate a first reinforcing layer, and then the resultant was immersed in a silicone solution again to form a second silicone layer. A silicone solution was applied onto a side portion of the formed second silicone layer to laminate a second reinforcing layer, and then the resultant was immersed in a silicone solution again to form a third silicone layer. As a result, a silicone shell in which a step was formed was manufactured. A filler was injected into the manufactured silicone shell to manufacture an artificial implant.

### Example 6

A breast-shaped mold was immersed in a silicone solution to form a first silicone layer. A silicone solution was applied onto a side portion of the formed first silicone layer to laminate a first reinforcing layer, and then the resultant was immersed in a silicone solution again to form a second silicone layer. A silicone solution was applied onto a side portion of the formed second silicone layer to laminate a second reinforcing layer, and then the resultant was immersed in a silicone solution again to form a third silicone layer. A silicone solution was applied onto a side portion of the formed third silicone layer to laminate a third reinforcing layer, and then the resultant was immersed in a silicone solution again to form a fourth silicone layer. As a result, a silicone shell in which a step was formed was manufactured. A filler was injected into the manufactured silicone shell to manufacture an artificial implant.

### Comparative Example

A breast-shaped mold was repeatedly immersed in a silicone solution three times to sequentially form a first silicone layer, a second silicone layer, and a third silicone layer with a uniform thickness of 0.5 mm, and thus a silicone shell was manufactured. A filler was injected into the manufactured silicone shell to manufacture an artificial implant.

### Experimental Example 1: Measurement of thickness of side portion

The number of reinforcing layer laminations of each artificial implant manufactured according to the examples and the comparative example and the thickness of the side portion according to the number of laminations were measured, and results thereof are shown in Table 1.

**[Table 1]**

| Classification | Number of reinforcing layer laminations | Thickness of side portion (mm) |
|---|---|---|
| Comparative Example | 0 | 0.5 |
| Example 1 | 1 | 0.53 |
| Example 2 | 2 | 0.56 |
| Example 3 | 3 | 0.59 |
| Example 4 | 1 | 0.54 |
| Example 5 | 2 | 0.57 |
| Example 6 | 3 | 0.60 |

Referring to Table 1, it can be confirmed that, as the number of reinforcing layer laminations increased, the side portion became thicker. Also, it can be confirmed that, although the lamination method of Examples 1 to 3 and the lamination method of Examples 4 to 6 were different (i.e., a method of laminating a reinforcing layer after forming a silicone layer and a method of alternately laminating a silicone layer and a reinforcing layer, respectively), when the number of reinforcing layer laminations was the same, the thickness of the side portion was similar.

### Experimental Example 2: Evaluation of properties of artificial implant

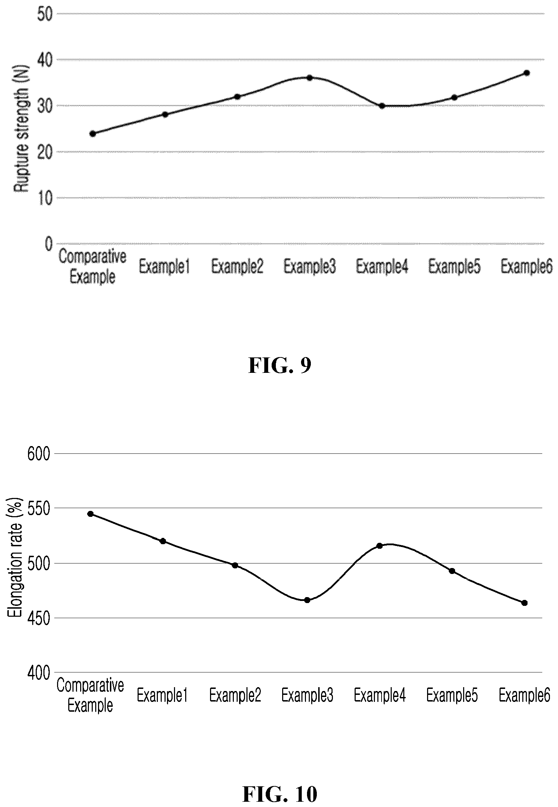

The rupture strength and elongation rate of each artificial implant manufactured according to the examples and the comparative example were evaluated, results thereof are shown in Table 2, and graphs showing the results of measuring the rupture strength and elongation rate are shown in FIGS. 9 and 10, respectively.

**[Table 2]**

| Classification | Rupture strength (N) | Elongation rate (%) |
|---|---|---|
| Comparative Example | 24 | 545 |
| Example 1 | 28 | 520 |
| Example 2 | 32 | 497 |
| Example 3 | 36 | 470 |
| Example 4 | 30 | 517 |
| Example 5 | 32 | 495 |
| Example 6 | 37 | 467 |

Referring to Table 2, it can be confirmed that the artificial implants of Examples 1 to 6 exhibited rupture strength values of 28 to 37 N, and as the number of reinforcing layer laminations increased, rupture strength values were improved. From the results, it can be seen that, as the thickness of the side portion is increased with the increased number of reinforcing layer laminations, durability is improved, and it can be expected to prevent side effects such as rupture, breakage, a rippling phenomenon, and the like upon insertion into the user's body and increase user satisfaction. On the other hand, in the case of the artificial implant of the comparative example, it can be confirmed that, since all portions were manufactured to have a uniform thickness, a rupture strength value was degraded compared to the examples.

In addition, it can be confirmed that the elongation rates of the artificial implants of Examples 1 to 6 decreased as the number of reinforcing layer laminations increased. From the results, it can be expected to continuously maintain a desired shape even when a filler is injected. On the other hand, the artificial implant of the comparative example exhibited the highest elongation rate of 545%. From the results, it can be expected that the artificial implant may be deformed over time, and accordingly, side effects such as the breakage and rupture of the artificial implant may occur.

The foregoing description of the specification is intended for illustration, and it will be understood by those skilled in the art to which the invention pertains that the invention can be easily modified in other specific forms without changing the technical spirit or essential features described in the specification. Therefore, it should be understood that the examples described above are only exemplary in all aspects and not limiting. For example, each of the constituents described as being one combined entity may be implemented separately, and similarly, constituents described as being separate entities may be implemented in a combined form.

It should be understood that the scope of the specification is defined by the following claims and that all changes or modifications derived from the meaning and scope of the claims and their equivalents are included in the scope of the specification.

### [List of Reference Numerals]

100: Artificial implant
110: Upper portion of silicone shell
120: Lower portion of silicone shell
130: Side portion of silicone shell
140: Reinforced structure
11, 12, 13, 14: First silicone layer, second silicone layer, third silicone layer, fourth silicone layer
21, 22, 23: First reinforcing layer, second reinforcing layer, third reinforcing layer

## Claims

1. An artificial implant comprising:
a silicone shell consisting of an upper portion, a lower portion, and a side portion; and
a filler injected into the silicone shell,
wherein the silicone shell includes one or more silicone layers and one or more reinforcing layers formed on at least a portion of a surface of the silicone layer, and
the reinforcing layer is provided in at least a portion of the lower portion and side portion of the silicone shell.

2. An artificial implant comprising:
a silicone shell consisting of an upper portion, a lower portion, and a side portion; and
a filler injected into the silicone shell,
wherein the silicone shell includes one or more silicone layers and one or more reinforcing layers formed on at least a portion of a surface of the silicone layer, and
the silicone shell has a layered structure in which a step is formed.

3. The artificial implant of claim 1, wherein at least a portion of the silicone shell is formed by continuously or alternately laminating the one or more silicone layers and the one or more reinforcing layers.

4. The artificial implant of claim 1, wherein the silicone shell has a thickness of 0.4 to 1.2 mm, and the reinforcing layer has a thickness of 0.01 to 0.3 mm.

5. The artificial implant of claim 2, wherein the upper portion and lower portion of the silicone shell have thicknesses of 0.4 to 0.8 mm, and the reinforced side portion of the silicone shell has a thickness of 0.4 to 1.2 mm.

6. The artificial implant of claim 1 or 2, wherein a portion of the silicone shell in which the reinforcing layer is provided satisfies at least one of the following conditions (i) and (ii):
(i) a rupture strength of 25 to 50 N; and
(ii) an elongation rate of 400 to 520%.

7. The artificial implant of claim 1 or 2, wherein a cross-section of the artificial implant has one shape selected from a circular shape, an oval shape, and a teardrop shape.

8. A method of manufacturing an artificial implant, comprising the steps of:
(a) immersing a breast-shaped mold in a silicone solution to form one or more silicone layers;
(b) applying a silicone solution onto at least a portion of the silicone layer to form one or more reinforcing layers;
(c) drying and curing the formed silicone layers and reinforcing layers to manufacture a silicone shell;
(d) forming an injection hole so that a filler is able to be injected into the silicone shell; and
(e) inputting the filler through the injection hole.

9. The method of claim 8, wherein in the step (b), the reinforcing layer is formed in at least a portion of a lower portion and side portion of the silicone shell.

10. The method of claim 8, wherein in the step (b), the reinforcing layer is formed so that the silicone shell has a layered structure having a step.

11. The method of claim 8, wherein the steps (a) and (b) are each repeatedly performed 1 to 10 times.

12. The method of claim 8, wherein the steps (a) and (b) are each alternately performed 1 to 10 times.
